# EUROPEAN PATENT SPECIFICATION

(11) **EP 2 796 104 B1**
(45) Date of publication and mention of the grant of the patent: **28.12.2016**
(21) Application number: 12860289.3
(22) Date of filing: 22.08.2012
(51) Int. Cl.: A61B 17/86, A61B 17/70, A61F 2/30

(54) **FUSION TYPE INTRASPINAL FIXING SCREW**
INTRASPINALE FUSIONSFIXATIONSSCHRAUBE
VIS DE FIXATION INTRARACHIDIENNE DE TYPE FUSION

(30) Priority: 19.12.2011 CN 201110427962
(43) Date of publication of application: 29.10.2014
(73) Proprietor: Beijing AK Medical Co., Ltd., Beijing 102200 (CN)
(72) Inventor: LIU, Zhongjun, Beijing 102200 (CN)
(74) Representative: Studio Torta S.p.A.
(86) International application number: PCT/CN2012/080474
(87) International publication number: WO 2013/091392

(56) References cited:
- WO-A1-02/24087
- WO-A1-2007/101267
- CN-A- 101 426 444
- CN-A- 102 123 674
- CN-A- 102 429 716
- CN-U- 2 095 643
- CN-U- 202 458 649
- US-A- 5 098 434
- US-A1- 2006 111 715
- US-A1- 2006 111 715

## Description

### Technical field of the invention

The disclosure relates to the field of spine internal fixation, in particular to a fusion spine internal fixation screw.

### Background of the invention

Spine is the central support of human body, with the upper part connected with the occipital bone at the bottom of skull and the lower part reaching the end of coccyx. Spine is connected with vertebrae, sacra and coccyx with the help of intervertebral discs, intervertebral joints and multiple ligaments so as to be the movement center of trunk and the hub for mechanical transmission and play the roles of supporting weight, absorbing shock, protecting the central nervous system, and providing the movement of flexion and extension forwards and backwards, lateral bending, twisting and so forth for the upper part of our body. Due to the lifestyle change and accelerated pace of life of modern people, the load on the spine and its surrounding tissues is increased, resulting in a higher damage chance and increasing the probability of diseases on spine. The common diseases related to spine, such as damage and loss of centrums caused by trauma, tumor, congenital malformation, tuberculosis, rheumatoid arthritis and other lesions, occur occasionally.

The spine internal fixation technology is becoming a conventional treatment means for spinal fractures, spondylolisthesis, spinal tumor, deformity correction and the like. A common spine internal fixation system includes a spine anterior and posterior screw-plate system, a screw-rod system, and an intervertebral and vertebral fusion system, and aims to provide early stability to promote the bony fusion of spine but has the risk of unscrewing and dislocating a screw in the long term. Most of the fixation systems adopt various metal bone screws, such as a steel plate screw and a pedicle screw, which are always provided with a thread bonded with sclerotins and a screw cap structure butted and connected with related components. The bone thread of the existing bone screw has a smooth surface and is easy to screw in and out, so as to be favourable for operation but increase the failure risk of the internal fixation system caused by loosening and unscrewing the screw in the later period.
Examples of internal fixation screws are disclosed in WO2007/1012671A1, US2006/0111715 A1, US 5098434 or CN 2095643 U.

### Summary of the invention

The present invention relates to a fusion spine internal fixation screw as claimed hereafter preferred embodiments of the invention are set forth in the dependent claims.

The objective of the disclosure is to provide a fusion spine internal fixation screw for preventing a screw from being loosened and unscrewed.

The fusion spine internal fixation screw provided by the disclosure comprises a screw cap section and a bone joint section connected with the screw cap section, wherein the bone joint section comprises a solid part and a porous fusion part fixedly arranged on a surface of the solid part; and the porous fusion part is provided with a porous structure inosculated with spine sclerotins ; the pourous structure is a multilayered porous structure, which comprises a plurality of pores communicated with one another.

Furthermore, the solid part comprises a solid reinforced core extended along an axial direction of the fusion spine internal fixation screw and a thread profile part spirally arranged around the solid reinforced core.

Furthermore, the porous fusion part is arranged in a thread pitch space of the thread profile part.

Furthermore, the porous fusion part is distributed at one end of the thread profile part far away from the screw cap section.

Furthermore, the solid reinforced core inside the porous fusion part is provided with fusion pores, which are pores penetrating one or more thread profiles along the axial direction of the fusion spine internal fixation screw.

Furthermore, a crest of the thread profile part protrudes from an outer surface of the fusion part.

Furthermore, an aperture of the porous structure is D, which is more than or equal to 50 microns and less than or equal to 1,200 microns.

Furthermore, the first end of the solid reinforced core is connected with the screw cap section and is tapered; and a bone ingrowth space is formed between the solid reinforced core and the thread profile part.

Furthermore, the thread profile part is wound on the solid reinforced core and is connected with the solid reinforced core at a root of its thread profile.

Furthermore, a surface of the bone joint part is completely or partially applied with a hydroxyapatite coating.

According to the fusion spine internal fixation screw of the disclosure, the porous fusion part of the bone joint section is of a porous structure, which can promote bone cells to grow therein to fuse sclerotins with the screw after the fusion spine internal fixation screw is implanted into the sclerotins, so as to finally form a bone fusion state, which can effectively prevent the fusion spine internal fixation screw from being loosened or unscrewed in a long term.

### Brief description of the drawings

The drawings constituting one part of the application are to provide further understanding of the disclosure, and the exemplary embodiments of the disclosure and the explanations thereof are intended to explain the disclosure, instead of improperly limiting the disclosure. In the drawings:
Fig. 1 is a front view showing the structure of a fusion spine internal fixation screw according to the first embodiment of the disclosure;
Fig. 2 is a front view showing the structure of a solid part of the fusion spine internal fixation screw in Fig. 1;
Fig. 3 is a perspective view showing the structure of a fusion spine internal fixation screw according to the second embodiment of the disclosure;
Fig. 4 is a lateral view showing the structure of a solid part of the fusion spine internal fixation screw in Fig. 3;
Fig. 5 is a front view showing the structure of a fusion spine internal fixation screw according to the third embodiment of the disclosure;
Fig. 6 is a perspective view showing the structure of a solid part of the fusion spine internal fixation screw in Fig. 5;
Fig. 7 is a perspective view showing the structure of a fusion spine internal fixation screw according to the fourth embodiment of the disclosure;
Fig. 8 is a perspective view showing the structure of a fusion spine internal fixation screw according to the fifth embodiment of the disclosure;
Fig. 9 is a perspective view showing the structure of a fusion spine internal fixation screw according to the sixth embodiment of the disclosure; and
Fig. 10 is a perspective view showing the structure of a porous structure layer of a fusion spine internal fixation screw according to the disclosure.

### Detailed description of the invention

The disclosure will be described below in detail with reference to the drawings and in conjunction with the embodiments.

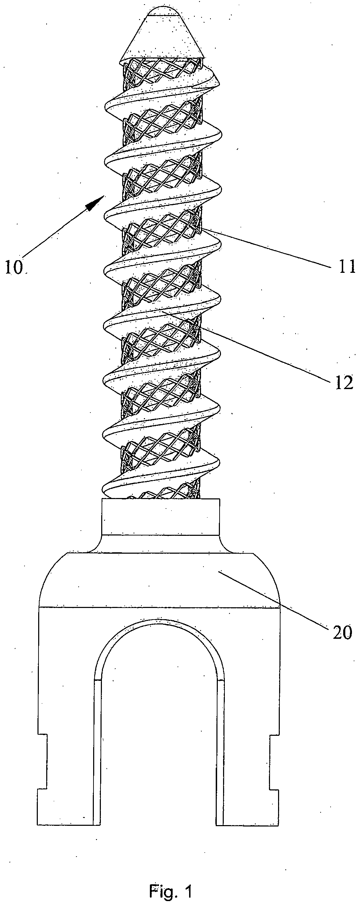

As shown in Fig. 1, the fusion spine internal fixation screw of the disclosure comprises a screw cap section 20 and a bone joint section 10 connected with the screw cap section 20, wherein the bone joint section 10 comprises a solid part 12 and a porous fusion part 11 fixedly arranged on the surface of the solid part 12; and the porous fusion part 11 is provided with a porous structure and inosculated with spine sclerotins.

To overcome the defects of the prior art, in the fusion spine internal fixation screw of the disclosure, the porous fusion part 11 of the bone joint section 10 inosculated with sclerotins is designed as a porous structure, which can promote bone cells to grow therein to fuse sclerotins with the screw after the fusion spine internal fixation screw is implanted into the sclerotins, so as to thoroughly prevent the screw from being loosened and unscrewed in the future.

According to the first embodiment of the disclosure, as shown in Fig. 1, a fusion spine internal fixation screw comprises a bone joint section 10 and a screw cap section 20. The fusion internal fixation screw is made of medical metals, so as to have good biocompatibility.

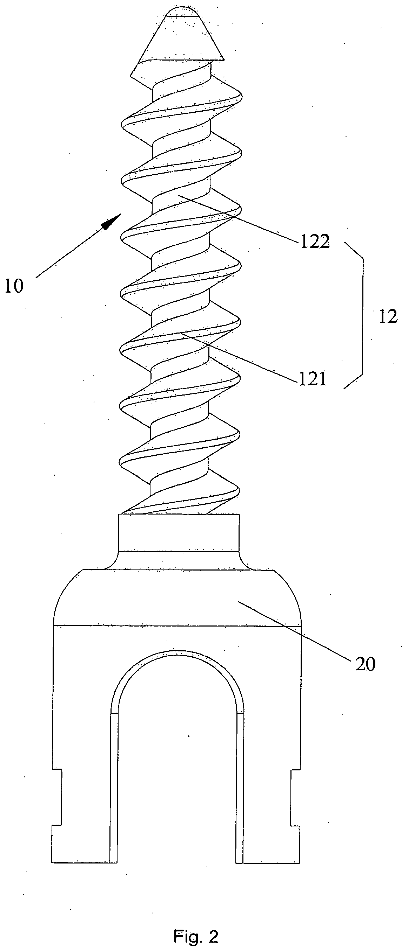

The bone joint section 10 is provided with a solid part 12 and a porous fusion part 11, wherein the solid part 12 is formed by solid metals and is located at the principal stress load area of the bone joint section 10. As shown in Fig. 2, the solid part 12 comprises a solid reinforced core 122 extended along the axial direction of the fixation screw and a thread profile part 121 spirally arranged around the solid reinforced core 122. Preferably, the solid reinforced core 122 of the solid part 12 is arranged at the axial center of the bone joint section 10; the core part of the thread profile part 121 is also a solid metal; the thread profile part 121 is spirally coiled around the solid reinforced core 122 and is connected with the solid reinforced core, so that the fusion spine internal fixation screw obtains enough mechanical strength and the elastic modules of the whole fusion spin internal fixation screw can be effectively adjusted to be matched with that of the cancellous bone and cortical bone of a human body, thereby creating a favorable condition for the bone growth and bone conduction of biomechanical load after an operation.

As shown in Fig. 1, the porous fusion part 11 is arranged in the thread pitch space of the thread profile part 121. The porous fusion part 11 is a multilayered porous structure, which comprises a plurality of pores communicated with one another.

The porous fusion part 11 is a porous structure layer outside the solid reinforced core 122 and in the thread pitch space of the thread profile part 121, and the porous structure layer is multiple layers of micropores, with surface communicated with its internal pores. The aperture of the porous structure is D, which is more than or equal to 50 microns and less than or equal to 1,200 microns. Channel and space for reconstructing capillaries are provided in the range of the aperture to create a good condition for the crawling and ingrowth of bone cells.

In the embodiment, preferably, as shown in Fig. 1, the space between the porous fusion part 11 and the solid part 12 is a transitional whole without obvious layers and borders so as to ensure the mechanical strength of the transitional layer.

The surface of the bone joint section 10 of the fusion spine internal fixation screw is completely or partially applied with hydroxyapatite coatings, which can induce the growth of bone cells.

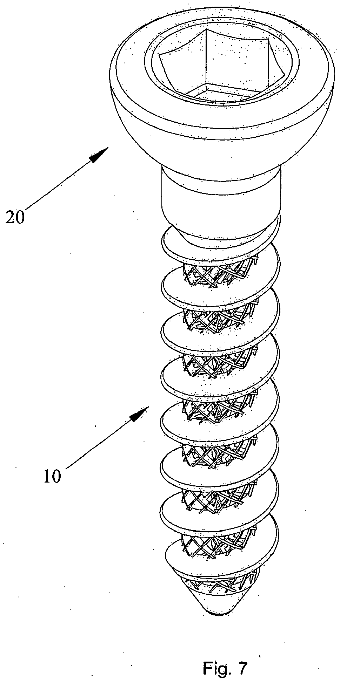

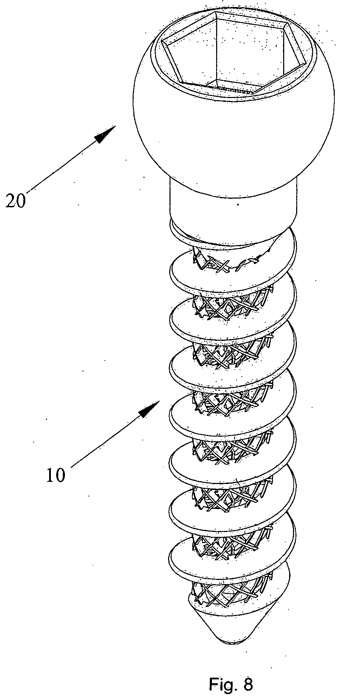

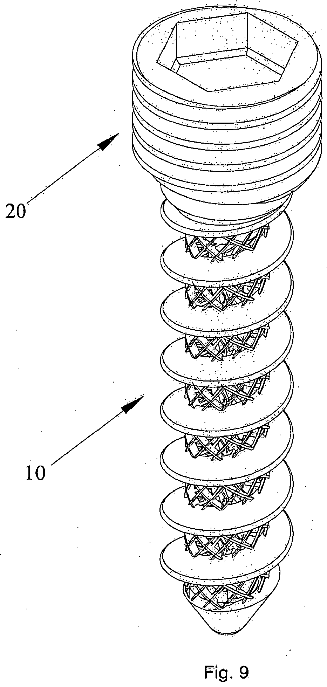

The screw cap section 20 of the fusion spine internal fixation screw is designed differently to meet the requirement of various fixation positions. For example, the screw cap sections 20 in the first, second and third embodiments in Figs. 1 to 6 are a fixation pedicle screw cap for fixing a screw rod; the screw cap section 20 in the fourth embodiment in Fig. 7 is a common plate fixation screw cap; the screw cap section 20 in the fifth embodiment in Fig. 8 is a reversing screw cap for fixing a screw rod; the screw cap section 20 in the sixth embodiment in Fig. 9 is a special plate fixation screw cap, and the screw cap section 20 is the pedicle screw cap of a non-fusion dynamical flexible fixation system (not shown) and the like, so as to adapt to various usages of the fusion spine internal fixation screw.

The bone joint section 10 of the disclosure can be in various forms.

As shown in Fig. 2, in the first embodiment of the disclosure, the thread profile part 121 is wound on the solid reinforced core 122 and is connected with the solid reinforced core 122 at the root of its thread profile. The crest of the thread profile part 121 protrudes from the outer surface of the porous fusion part 11. For such fixation screw, its thread profile part 121 and solid reinforced core 122 are integrated into a whole, so that the fusion spine internal fixation screw in the embodiment has enough mechanical strength.

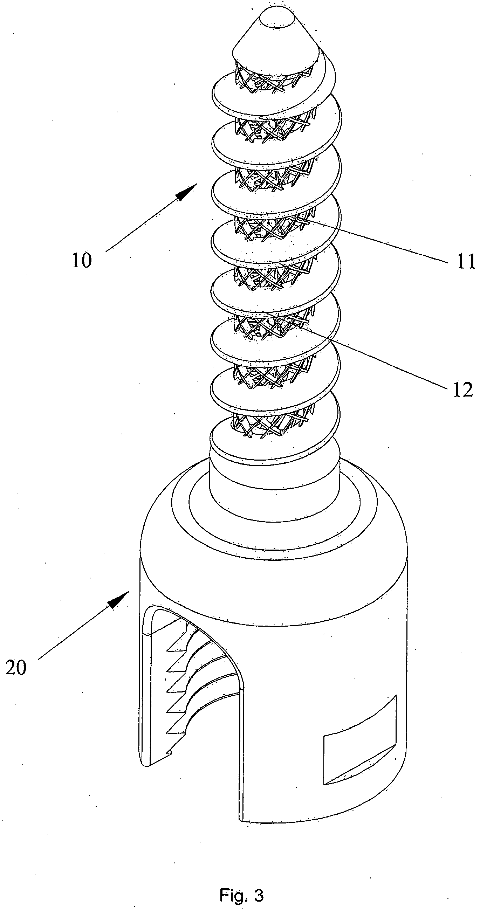

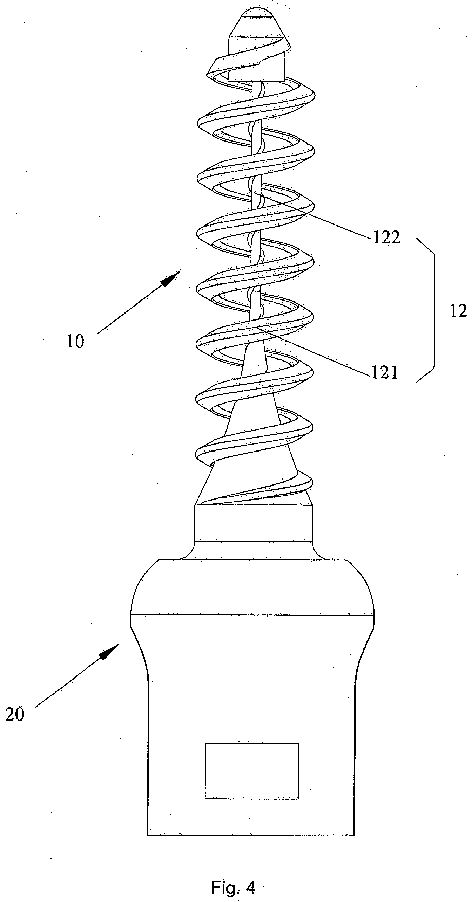

As shown in Figs. 3 and 4, according to the second embodiment of the disclosure, the first end of the solid reinforced core 122 is connected with the screw cap section 20 and is tapered; and a bone ingrowth space is reserved between the solid reinforced core 122 and the thread profile part 121. In the embodiment, only the end of the thread profile part 121 adjacent to the screw cap section 20 is connected with the solid reinforced core 122; and its middle part and the other end are both wound around the solid reinforced core 122 without contacting the solid reinforced core 122. Such bone joint section 10 has more bone ingrowth space than that of the structure shown in Fig. 2 and is also lighter.

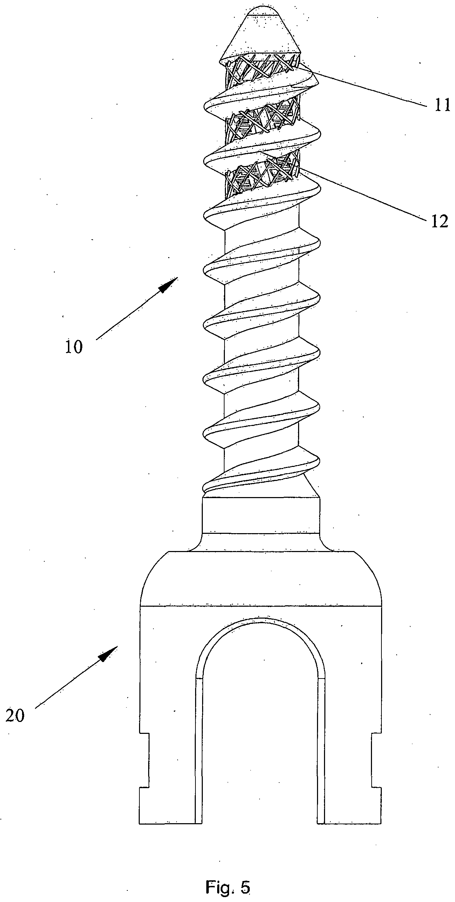

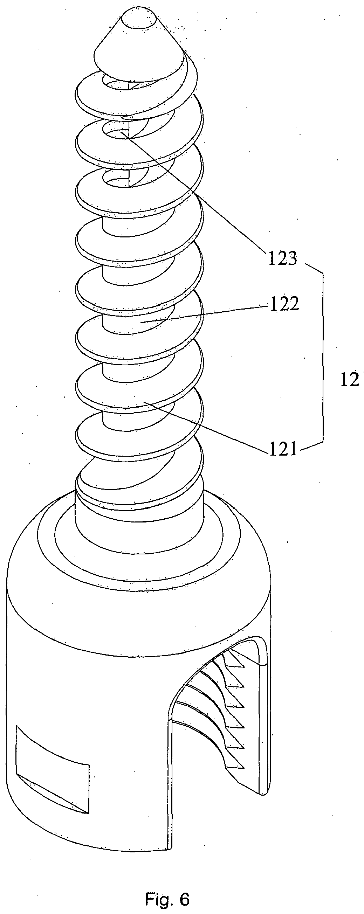

As shown in Figs. 5 and 6, according to the third embodiment of the disclosure, the porous fusion part 11 is distributed at the end of the thread profile part 121 far away from the screw cap section 20. The solid reinforced core 122 inside the porous fusion part 11 is provided with fusion pores, which are holes penetrating one or more thread profiles along the axial direction of the fixation screw. In the embodiment, the porous fusion part 11 is only distributed at one end of the screw cap section 20; and the solid reinforced core 122 is provided with fusion pores 123 for the ingrowth of bone. Such bone joint section 10 is provided with a porous fusion part 11 only at the end far away from the screw cap section 20; and the thread profile part 121 adjacent to the screw cap section 20 is still formed by solid metals, so that the maximally-stressed thread profile part 121 adjacent to the screw cap section 20 can be maintained in a better mechanical state.

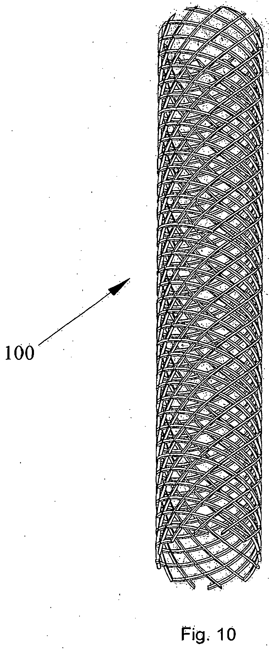

The fusion spine internal fixation screw of the disclosure is made of medical metals with good biocompatibility; and the porous structure layer is provided with a plurality of pores and channels which are communicated with one another on surface and inside, and the porous structure layer is tightly contacted with sclerotins to facilitate the ingrowth of bone cells, thereby obtaining long-term stability, with the structure 100 as shown in Fig. 10. In the disclosure, the porous structure layer is melt by the laser or high-energy electronic beam rapid forming technology, as follows:
1, the 3D data model of the porous structure layer is designed and constructed in a computer;
2, the 3D data model is layered by professional software to obtain the outline data of a series of single-layer slices;
3, the data of the series of slices is input to the laser or high-energy electronic beam fast forming equipment;
4, metal or ceramics powder, the thickness of which corresponds to the layering height of the 3D data model, is laid in the processing chamber of the laser or high-energy electronic beam fast forming equipment;
5, the metal or ceramic powder is scanned or selectively melt by the laser or high-energy electronic beam under the control of a computer;
6, the steps of laying and scanning and melting the powder are repeated to sinter the materials selected to be melt in each layer into a whole; and
7, after the melting is completed on all layers, the powder not molten is removed to obtain the porous structure layer in the required shape.

The porous fusion part and the solid part are obtained by fusion by the laser or high-energy electronic beam rapid forming technology; then a corresponding screw cap section and the mating structure of other parts are obtained by lathes, milling machines and other common mechanic cutting processing methods; and the bone joint section is processed mechanically or not according to the design needs, wherein the process is arranged rationally and very easy to implement.

According to the fusion spine internal fixation screw of the disclosure, the porous fusion part of the bone joint section is of a porous structure, which can promote bone cells to grow therein to fuse sclerotins with the screw after the fusion spine internal fixation screw is implanted into the sclerotins, so as to finally form a bone fusion state, which can effectively prevent the fusion spine internal fixation screw from being loosened or unscrewed in a long term.

## Claims

1. A fusion spine internal fixation screw, comprising a screw cap section (20) and a bone joint section (10) connected with the screw cap section (20), the bone joint section (10) comprises a solid part (12) and a porous fusion part (11) fixedly arranged on a surface of the solid part (12); and the porous fusion part (11) is provided with a porous structure inosculated with spine sclerotins, **characterized in that** the porous fusion part (11) is a multilayered porous structure, which comprises a plurality of pores communicated with one another.

2. The fusion spine internal fixation screw according to claim 1, **characterized in that**, the solid part (12) comprises a solid reinforced core (122) extended along an axial direction of the fusion spine internal fixation screw and a thread profile part (121) spirally arranged around the solid reinforced core (122).

3. The fusion spine internal fixation screw according to claim 2, **characterized in that**, the porous fusion part (11) is arranged in a thread pitch space of the thread profile part (121).

4. The fusion spine internal fixation screw according to claim 3, **characterized in that**, the porous fusion part (11) is distributed at one end of the thread profile part (121) far away from the screw cap section (20).

5. The fusion spine internal fixation screw according to claim 4, **characterized in that**, the solid reinforced core (122) inside the porous fusion part (11) is provided with fusion pores, which are pores penetrating one or more thread profiles along the axial direction of the fusion spine internal fixation screw.

6. The fusion spine internal fixation screw according to claim 2, **characterized in that**, a crest of the thread profile part (121) protrudes from an outer surface of the fusion part (11).

7. The fusion spine internal fixation screw according to claim 1, **characterized in that**, an aperture of the porous structure is D, which is more than or equal to 50 microns and less than or equal to 1,200 microns.

8. The fusion spine internal fixation screw according to claim 2, **characterized in that**, the first end of the solid reinforced core (122) is connected with the screw cap section (20) and is tapered; and a bone ingrowth space is formed between the solid reinforced core (122) and the thread profile part (121).

9. The fusion spine internal fixation screw according to claim 2, **characterized in that**, the thread profile part (121) is wound on the solid reinforced core (122) and is connected with the solid reinforced core (122) at a root of its thread profile.

10. The fusion spine internal fixation screw according to any one of claims 1 to 9, **characterized in that**, a surface of the bone joint part (10) is completely or partially applied with hydroxyapatite

## Patentansprüche

1. Intraspinale Fusionsfixationsschraube, umfassend einen Schraubsockelbereich (20) und einen Knochenverbindungsbereich (10), der mit dem Schraubsockelbereich (20) verbunden ist, wobei der Knochenverbindungsbereich (10) einen massiven Teil (12) und einen durchlässigen Fusionsteil (11), der fest an einer Oberfläche von dem massiven Teil (12) angeordnet ist, umfasst; und wobei der durchlässige Fusionsteil (11) mit einer durchlässigen Struktur vorgesehen ist, die mit Wirbelsäulen-Sklerotinen verflochten ist, **dadurch gekennzeichnet, dass** der durchlässige Fusionsteil (11) eine vielschichtige, durchlässige Struktur ist, die eine Vielzahl von Poren umfasset, die miteinander kommunizieren können.

2. Intraspinale Fusionsfixationsschraube nach Anspruch 1, **dadurch gekennzeichnet, dass** der massive Teil (12) einen massiven, verstärkten Kern (122) umfasst, der sich entlang einer axialen Richtung von der intraspinalen Fusionsfixationsschraube erstreckt, und dass ein Gewindeprofilteil (121) spiralförmig um den massiven, verstärkten Kern (122) angeordnet ist.

3. Intraspinale Fusionsfixationsschraube nach Anspruch 2, **dadurch gekennzeichnet, dass** der durchlässige Fusionsteil (11) in einem Gewindesteigungsraum von dem Gewindeprofilteil (121) angeordnet ist.

4. Intraspinale Fusionsfixationsschraube nach Anspruch 3, **dadurch gekennzeichnet, dass** der durchlässige Fusionsteil (11) an einem Ende von dem Gewindeprofilteil (121) weit entfernt von dem Schraubsockelbereich (20) verteilt ist.

5. Intraspinale Fusionsfixationsschraube nach Anspruch 4, **dadurch gekennzeichnet, dass** der massive, verstärkte Kern (122) innerhalb dem durchlässigen Fusionsteil (11) mit Fusionsporen vorgesehen ist, welche Poren sind, die ein oder mehrere Gewindeprofile entlang der axialen Richtung von der intraspinalen Fusionsfixationsschraube durchdringen.

6. Intraspinale Fusionsfixationsschraube nach Anspruch 2, **dadurch gekennzeichnet, dass** ein Scheitelpunkt des Gewindeprofilteils (121) von einer Außenfläche von dem Fusionsteil (11) herausragt.

7. Intraspinale Fusionsfixationsschraube nach Anspruch 1, **dadurch gekennzeichnet, dass** eine Öffnung von der durchlässigen Struktur D ist, die größer oder gleich groß ist wie 50 Mikrons und kleiner oder gleich groß wie 1200 Mikrons ist.

8. Intraspinale Fusionsfixationsschraube nach Anspruch 2, **dadurch gekennzeichnet, dass** das erste Ende von dem massiven, verstärkten Kern (122) mit dem Schraubsockelbereich (20) verbunden ist und konisch ist; und ein Raum ausgebildet wird, zum Einwachsen des Knochens zwischen dem massiven, verstärkten Kern (122) und dem Gewindeprofilteil (121).

9. Intraspinale Fusionsfixationsschraube nach Anspruch 2, **dadurch gekennzeichnet, dass** der Gewindeprofilteil (121) an dem massiven, verstärkten Kern (122) gewunden ist und mit dem massiven, verstärkten Kern (122) an einem Ursprung von seinem Gewindeprofil verbunden ist.

10. Intraspinale Fusionsfixationsschraube nach einem beliebigen der Ansprüche 1 bis 9, **dadurch gekennzeichnet, dass** eine Oberfläche von dem Knochenverbindungsteil (10) vollständig oder teilweise mit Hydroxylapatit verwendet wird.

## Revendications

1. Vis de fixation intrarachidienne de type fusion, comportant une portion (20) tête de vis et une portion (10) de jonction osseuse reliée à la portion (20) tête de vis, la portion (10) de jonction osseuse comporte une partie (12) pleine et une partie (11) de fusion poreuse agencée de manière fixe sur une surface de la partie (12) pleine ; et la partie (11) de fusion poreuse est pourvue d'une structure poreuse intimement liée à des sclérotines rachidiennes,
**caractérisée en ce que** la partie (11) de fusion poreuse est une structure poreuse multicouche, qui comporte une pluralité de pores communiquant les uns avec les autres.

2. Vis de fixation intrarachidienne de type fusion selon la revendication 1, **caractérisée en ce que** la partie (12) pleine comporte un noyau (122) renforcé plein qui s'étend le long d'une direction axiale de la vis de fixation intrarachidienne de fusion et une partie (121) profil de filetage disposée en spirale autour du noyau (122) renforcé plein.

3. Vis de fixation intrarachidienne de type fusion selon la revendication 2, **caractérisée en ce que** la partie (11) de fusion poreuse est agencée dans un espace entre des filets de la partie (121) profil de filetage.

4. Vis de fixation intrarachidienne de type fusion selon la revendication 3, **caractérisée en ce que** la partie (11) de fusion poreuse est distribuée à une extrémité de la partie (121) profil de filetage loin de la portion (20) tête de vis.

5. Vis de fixation intrarachidienne de type fusion selon la revendication 4, **caractérisée en ce que** le noyau (122) renforcé plein à l'intérieur de la partie (11) de fusion poreuse est pourvu de pores de fusion, qui sont des pores pénétrant dans un ou plusieurs profils de filetage le long de la direction axiale de la vis de fixation interne rachidienne de fusion.

6. Vis de fixation intrarachidienne de type fusion selon la revendication 2, **caractérisée en ce qu'**une crête de la partie (121) profil de filetage fait saillie depuis une surface externe de la partie (11) de fusion.

7. Vis de fixation intrarachidienne de type fusion selon la revendication 1, **caractérisée en ce qu'**une ouverture de la structure poreuse est D, qui est supérieure ou égale à 50 microns et inférieure ou égale à 1200 microns.

8. Vis de fixation intrarachidienne de type fusion selon la revendication 2, **caractérisée en ce que** la première extrémité du noyau (122) renforcé plein est reliée à la portion (20) tête de vis et est conique ; et un espace de croissance osseuse est formé entre le noyau (122) renforcé plein et la partie (121) profil de filetage.

9. Vis de fixation intrarachidienne de type fusion selon la revendication 2, **caractérisée en ce que** la partie (121) profil de filetage est enroulée sur le noyau (122) renforcé plein et est reliée au noyau (122) renforcé plein au niveau d'une base de son profil de filetage.

10. Vis de fixation intrarachidienne de type fusion selon l'une quelconque des revendications 1 à 9, **caractérisée en ce qu'**une surface de la portion (10) de jonction osseuse est complètement ou partiellement revêtue d'hydroxyapatite.
